(19) Europäisches Patentamt
European Patent Office
Office européen des brevets

(11) Veröffentlichungsnummer: **0 302 441 B1**

(12) **EUROPÄISCHE PATENTSCHRIFT**

(45) Veröffentlichungstag der Patentschrift: **12.05.93**

(51) Int. Cl.5: **C07C 317/14**, C07C 311/15, C07C 309/32, C08K 5/41

(21) Anmeldenummer: **88112508.2**

(22) Anmeldetag: **02.08.88**

# (54) Bis-styrylverbindungen.

(30) Priorität: **05.08.87 DE 3725928**
**21.04.88 DE 3813334**

(43) Veröffentlichungstag der Anmeldung:
**08.02.89 Patentblatt 89/06**

(45) Bekanntmachung des Hinweises auf die Patenterteilung:
**12.05.93 Patentblatt 93/19**

(84) Benannte Vertragsstaaten:
**CH DE FR GB IT LI**

(56) Entgegenhaltungen:
**EP-A- 0 019 078**
**EP-A- 0 032 254**
**EP-A- 0 252 009**
**DE-A- 2 209 223**
**DE-A- 2 401 665**

(73) Patentinhaber: **BASF Aktiengesellschaft**
**Carl-Bosch-Strasse 38**
**W-6700 Ludwigshafen(DE)**

(72) Erfinder: **Etzbach, Karl-Heinz, Dr.**
**Carl-Bosch-Ring 55**
**W-6710 Frankenthal(DE)**
Erfinder: **Hauptreif, Manfred, Dr.**
**Mundenheimer Strasse 167**
**W-6700 Ludwigshafen(DE)**
Erfinder: **Sens, Ruediger, Dr.**
**Medicusstrasse 12**
**W-6800 Mannheim 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Bis-styrylverbindungen, die Sulfonylgruppen enthaltende Substituenten aufweisen sowie ihre Verwendung zum Aufhellen von Polyester.

Aus der EP-A-252 009 sind optische Aufheller auf Basis von Bis-styrylderivaten bekannt, die über einen Phenoxysulfonylrest oder über verschiedene Alkylsulfonylreste verfügen. Folgende Bis-styrylderivate sind dort beschrieben

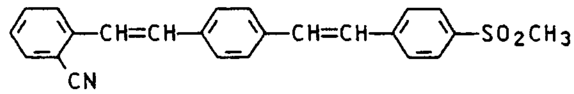

oder $-SO_2C_2H_5$;

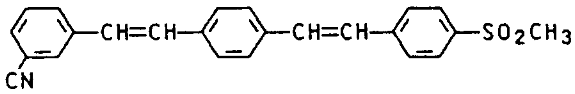

und

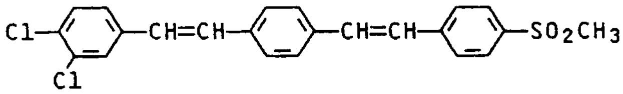

Aufgabe der vorliegenden Erfindung war es, neue Bis-styrylverbindungen bereitzustellen, die vorteilhafte anwendungstechnische Eigenschaften als optische Aufheller aufweisen.

Es wurden nun Bis-styrylverbindungen der Formel I

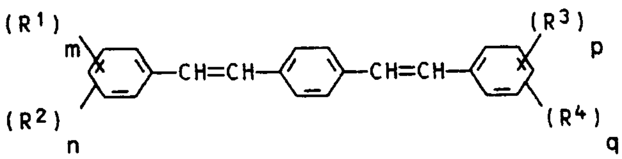

(I)

gefunden, in der

| | |
|---|---|
| $R^1$ | gegebenenfalls substituiertes Phenoxysulfonyl, $C_1-C_{13}$-Alkylsulfonyl, das gegebenenfalls durch Hydroxy oder $C_1-C_4$-Alkanoyloxy substituiert ist und dessen Alkylkette durch ein oder mehrere Sauerstoffatome unterbrochen sein kann, $C_1-C_8$-Mono- oder Dialkylsulfamoyl, wobei die Alkylkette jeweils durch ein oder mehrere Sauerstoffatome unterbrochen sein kann, oder gegebenenfalls substituiertes Phenylsulfonyl, |
| $R^2$ und $R^3$ | gleich oder verschieden sind und jeweils unabhängig voneinander Wasserstoff, Fluor, Chlor, $C_1-C_4$-Alkyl, $C_1-C_4$-Alkoxy oder gegebenenfalls substituiertes Phenoxy, |
| $R^4$ | Fluor, Chlor, Cyano, Carbamoyl, $C_1-C_8$-Mono- oder -Dialkylcarbamoyl, deren Alkylkette jeweils durch ein oder mehrere Sauerstoffatome unterbrochen sein kann, Pyrrolidinocarbonyl, Piperidinocarbonyl, Morpholinocarbonyl, Piperazinocarbonyl, N-($C_1-C_4$-Alkyl)piperazinocarbonyl, $C_1-C_8$-Alkoxycarbonyl, dessen Alkylkette durch ein oder mehrere Sauerstoffatome unterbrochen sein kann, oder gegebenenfalls substituiertes Phenoxycarbonyl und |
| m, n, p und q | gleich oder verschieden sind und jeweils unabhängig voneinander 1 oder 2 bedeuten, wobei die Verbindungen der Formeln |

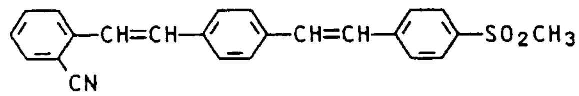

,

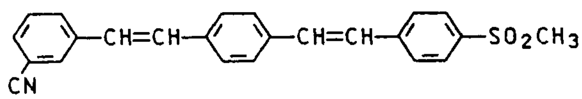

und

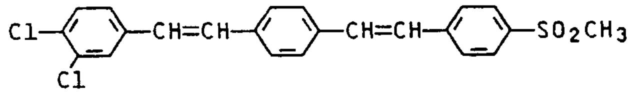

ausgenommen sind.

Alle in der obengenannten Formel I auftretenden Alkylreste können sowohl geradkettig als auch verzweigt sein.

Für den Fall, daß in den neuen Bis-styrylverbindungen der Formel I Alkylketten auftreten, die durch Sauerstoffatome unterbrochen sind, sind, sofern nicht anders vermerkt, solche Verbindungen bevorzugt, bei denen die Alkylketten durch ein, zwei oder drei Sauerstoffatome unterbrochen sind.

Für den Fall, daß die in der obengenannten Formel I auftretenden Phenylreste substituiert sind, kommen, sofern nicht anders vermerkt, als Substituenten z.B. $C_1-C_4$-Alkyl, $C_1-C_4$-Alkoxy oder Halogen, insbesondere Fluor oder Chlor, in Betracht.

Wenn in der obengenannten Formel I der Substituent $R^1$ für substituiertes Phenoxysulfonyl steht, kommen als Substituenten am Phenylring z.B. $C_1-C_{10}$-Alkyl, das gegebenenfalls durch ein, zwei oder drei Sauerstoffatome unterbrochen ist, $C_1-C_{10}$-Alkoxy, das gegebenenfalls durch ein, zwei oder drei Sauerstoffatome unterbrochen ist, oder Halogen, insbesondere Fluor oder Chlor in Betracht.

$R^1$ steht beispielsweise für Phenoxysulfonyl, 4-Methylphenoxysulfonyl, 2,4-Dimethylphenoxysulfonyl, 4-Ethylphenoxysulfonyl, 4-Propylphenoxysulfonyl, 4-Butylphenoxysulfonyl, 4-Pentylphenoxysulfonyl, 4-Hexylphenoxysulfonyl, 4-(2-Ethylhexyl)phenoxysulfonyl, 4-Nonylphenoxysulfonyl, 4-Decylphenoxysulfonyl, 4-(2-Methoxyethyl)phenoxysulfonyl, 4-(2-Ethoxyethyl)phenoxysulfonyl, 4-(2-Propoxyethyl)phenoxysulfonyl, 4-(2-Butoxyethyl)phenoxysulfonyl, 4-(3,6-Dioxaheptyl)phenoxysulfonyl, 4-(3,6-Dioxaoctyl)phenoxysulfonyl, 4-(3,6,9-Trioxadecyl)phenoxysulfonyl, 2-Methoxyphenoxysulfonyl, 4-Methoxyphenoxysulfonyl, 4-Ethoxyphenoxysulfonyl, 4-Isopropoxyphenoxysulfonyl, 4-Butoxyphenoxysulfonyl, 4-Hexyloxyphenoxysulfonyl, 4-Heptyloxyphenoxysulfonyl, 4-Octyloxyphenoxysulfonyl, 4-(2-Ethylhexyloxy)phenoxysulfonyl, 4-Decyloxyphenoxysulfonyl, 4-(2-Methoxyethoxy)phenoxysulfonyl, 4-(2-Ethoxyethoxy)phenoxysulfonyl, 4-(3,6-Dioxaheptyloxy)phenoxysulfonyl,4-(3,6-Dioxaoctyloxy)-phenoxysulfonyl, 4-(3,6,9-Trioxadecyloxy)phenoxysulfonyl, 4-Fluorphenoxysulfonyl, 2-Chlorphenoxysulfonyl, 2,4-Dichlorphenoxysulfonyl, Methylsulfonyl, Ethylsulfonyl, Propylsulfonyl, Isopropylsulfonyl, Butylsulfonyl, Isobutylsulfonyl, sec-Butylsulfonyl, Pentylsulfonyl, Isopentylsulfonyl, Neopentylsulfonyl, tert-Pentylsulfonyl, Hexylsulfonyl, Heptylsulfonyl, Octylsulfonyl, Isooctylsulfonyl, 2-Ethylhexylsulfonyl, Nonylsulfonyl, Isononylsulfonyl, Decylsulfonyl, Isodecylsulfonyl, Undecylsulfonyl, Dodecylsulfonyl, Tridecylsulfonyl, Isotridecylsulfonyl (die Bezeichnungen Isooctyl, Isononyl, Isodecyl und Isotridecyl sind Trivialbezeichnungen und stammen von den nach der Oxosynthese erhaltenen Alkoholen [vgl. dazu Ullmanns Enzyklopädie der technischen Chemie, 4. Auflage, Band 7, Seiten 215 bis 217 sowie Band 11, Seiten 435 und 436]), 2-Hydroxyethylsulfonyl,2- oder 3-Hydroxypropylsulfonyl, 2- oder 4-Hydroxybutylsulfonyl, 5-Hydroxypentylsulfonyl, 5-Hydroxy-2-oxapentylsulfonyl, 6-Hydroxyhexylsulfonyl, 2-Formyloxyethylsulfonyl, 2-Acetyloxyethylsulfonyl, 2-Propionyloxyethylsulfonyl, 2-Butyryloxyethylsulfonyl, 2- oder 3-Acetyloxypropylsulfonyl, 2- oder 4-Acetyloxybutylsulfonyl, 5-Propionyloxy-2-oxapentylsulfonyl, 2-Methoxyethylsulfonyl, 2-Ethoxyethylsulfonyl, 2-Propoxyethylsulfonyl, 2-Butoxyethylsulfonyl, 2- oder 3-Methoxypropylsulfonyl, 2- oder 4-Methoxybutylsulfonyl, 3,6-Dioxaheptylsulfonyl, 3,6-Dioxaoctylsulfonyl, 3,6-Dioxadecylsulfonyl, 3,6,9-Trioxadecylsulfonyl, 4-Oxa-6-ethyldecylsulfonyl, 3,6,9,12-Tetraoxatridecylsulfonyl, 5,10,15-Trioxahexadecylsulfonyl, Mono- oder Dimethylsulfamoyl, Mono- oder Dipropylsulfamoyl, Mono- oder Dibutylsulfamoyl, Mono- oder Di(2-ethylhexyl)sulfamoyl, Mono- oder Di(2-methoxyethyl)sulfamoyl, Mono- oder Di-(2-ethoxyethyl)sulfamoyl, Mono- oder Di(2-propoxyethyl)sulfamoyl, Mono- oder Di(2-butoxyethyl)sulfamoyl, Mono- oder Di(3-methoxypropyl)sulfamoyl, Mono- oder Di(3-ethoxypropyl)sulfamoyl, Phenylsulfonyl, 4-Methylphenylsulfonyl, 4-Methoxyphenylsulfonyl, 2-Ethoxyphenylsulfonyl, 4-Fluorphenylsulfonyl, 4-Chlorphenylsulfonyl, 2,4-Dichlorphenylsulfonyl oder 2,6-Dichlorphenylsulfonyl.

$R^2$ und $R^3$ stehen beispielsweise für Methyl, Ethyl, Propyl, Isopropyl, Butyl, Isobutyl, sec-Butyl, tert-Butyl, Methoxy, Ethoxy, Propoxy, Isopropoxy, Butoxy, Isobutoxy, sec-Butoxy, Phenoxy, 2-Methylphenoxy, 4-Methylphenoxy, 4-Methoxyphenoxy, 4-Fluorphenoxy oder 4-Chlorphenoxy.

$R^4$ steht beispielsweise für Methylcarbamoyl, Ethylcarbamoyl, Propylcarbamoyl, Isopropylcarbamoyl, Butylcarbamoyl, Isobutylcarbamoyl, Pentylcarbamoyl, Isopentylcarbamoyl, sec-Pentylcarbamoyl, Hexylcarbamoyl, Heptylcarbamoyl, Octylcarbamoyl, 2-Ethylhexylcarbamoyl, Dimethylcarbamoyl, Diethylcarbamoyl, Dipropylcarbamoyl, Diisopropylcarbamoyl, Dibutylcarbamoyl, Di-(2-ethylhexyl)-carbamoyl, N-Methyl-N-ethylcarbamoyl, 2-Methoxyethylcarbamoyl, 2-Ethoxyethylcarbamoyl, 3,6-Dioxaheptylcarbamoyl, 3,6-Dioxaoctylcarbamoyl, Bis-(2-methoxyethyl)-carbamoyl, Bis-(2-ethoxyethyl)-carbamoyl, Bis-(3,6-dioxaheptyl)-carbamoyl, Bis-(3,6-dioxaoctyl)-carbamoyl, N-Methylpiperazinocarbonyl, N-Ethylpiperazinocarbonyl, Methoxycarbonyl, Ethoxycarbonyl, Propoxycarbonyl, Isopropoxycarbonyl, Butoxycarbonyl, Isobutoxycarbonyl, Pentyloxycarbonyl, Isopentyloxycarbonyl, sec-Pentyloxycarbonyl, tert-Pentyloxycarbonyl, Hexyloxycarbonyl, Heptyloxycarbonyl, Octyloxycarbonyl, 2-Ethylhexyloxycarbonyl, 2-Methoxyethoxycarbonyl, 2-Ethoxyethoxycarbonyl, 3,6-Dioxaheptyloxycarbonyl, 3,6-Dioxaoctyloxycarbonyl, Phenoxycarbonyl, 4-Methylphenoxycarbonyl, 2-Methoxyphenoxycarbonyl, 3-Methoxyphenoxycarbonyl, 4-Fluorphenoxycarbonyl oder 4-Chlorphenoxycarbonyl.

Bevorzugt sind Bis-styrylverbindungen der Formel I, in der $R^1$ Phenoxysulfonyl, durch $C_1-C_6$-Alkoxy substituiertes Phenoxysulfonyl, $C_2-C_{10}$-Alkylsulfonyl, dessen Alkylkette durch ein oder zwei Sauerstoffatome unterbrochen ist und das gegebenenfalls durch Hydroxy oder $C_1-C_4$-Alkanoyloxy substituiert ist, $C_1-C_8$-Mono- oder Dialkylsulfoamoyl, wobei die Alkylkette jeweils durch ein oder zwei Sauerstoffatome unterbrochen sein kann, oder Phenylsulfonyl, $R^2$ und $R^3$ jeweils Wasserstoff, $R^4$ Chlor, Cyano, $C_1-C_4$-Alkoxycarbonyl, $C_1-C_4$-Dialkylcarbamoyl, Phenoxycarbonyl oder durch Methyl, Ethyl, Methoxy oder Chlor substituiertes Phenoxycarbonyl, und m und q jeweils 1 bedeuten.

Die erfindungsgemäßen Bis-styrylverbindungen werden vorteilhaft durch eine Wittig-Horner-Reaktion erhalten. Dazu setzt man beispielsweise zunächst in einer ersten Stufe Terephthalaldehyd mit einem Benzylphosphonsäureester der Formel II

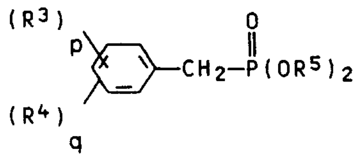 (II),

in der $R^3$, $R^4$, p und q jeweils die obengenannte Bedeutung besitzen und $R^5$ für $C_1-C_4$-Alkyl steht, im Molverhältnis von 1:1 in Gegenwart von 1 bis 1,1 Mol, jeweils bezogen auf 1 Mol Benzylphosphonsäureester II, einer Base und in Gegenwart eines inerten Lösungsmittels um.

Als Base dient z.B. Alkalialkanolat, z.B. Natrium- oder Kaliummethanolat, -ethanolat, -propanolat, -isopropanolat oder -butanolat. Vorzugsweise verwendet man Natriummethanolat oder -ethanolat.

Geeignete Lösungsmittel sind beispielsweise Ester, Ether oder gegebenenfalls substituierte Kohlenwasserstoffe, wie Ethylacetat, Propylacetat, Isopropylacetat, Butylacetat, Isobutylacetat, 2-Methoxyethylacetat, Diethylenglykoldimethylether, Tetrahydrofuran, Dioxan, Toluol, Xylol, Chlorbenzol oder o-Dichlorbenzol.

Die Verwendung von Ethylacetat, Butylacetat, Isobutylacetat, 2-Methoxyethylacetat, Tetrahydrofuran oder Dioxan ist dabei bevorzugt.

Die Umsetzung, die zu einem Formylstilbenderivat der Formel III

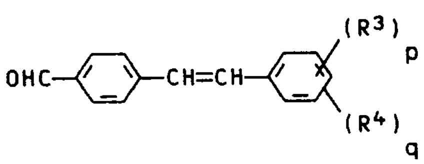 (III)

führt, in der $R^3$, $R^4$, p und q jeweils die obengenannte Bedeutung besitzen, wird zweckmäßig bei einer Temperatur von 20 bis 50°C durchgeführt. Man legt dabei Terephthalaldehyd und Benzylphosphonester zusammen mit dem Lösungsmittel vor und gibt die Base, vorteilhaft in Form der jeweiligen alkanolischen

Lösung langsam zu.

Nach etwa 6 bis 18 Stunden ist die Umsetzung beendet. Man trennt dann das Formylstilbenderivat III ab, wäscht es mit Wasser nach und trocknet es.

Anschließend wird in einer zweiten Stufe das Formylstilben III durch Umsetzung mit einem Benzyl-phosphonester der Formel IV

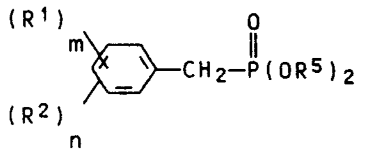

(IV),

in der $R^1$, $R^2$, $R^5$, m und n jeweils die obengenannte Bedeutung besitzen, in die Bis-styrylverbindung I übergeführt.

Die Reaktionsbedingungen für die zweite Stufe sind im allgemeinen denen der ersten Stufe sehr ähnlich. Ein Unterschied besteht in der Wahl des Lösungsmittels. Es ist von Vorteil, die Umsetzung von Formylstilben III und Benzylphosphonester IV in Carbonsäureamiden als Lösungsmittel durchzuführen. Besonders geeignet sind dabei N,N-Dimethylformamid, N,N-Diethylformamid oder N-Methylpyrrolidon.

Die erfindungsgemäßen Bis-styrylverbindungen der Formel I eignen sich in vorteilhafter Weise zum optischen Aufhellen von synthetischen, halbsynthetischen oder natürlichen organischen Materialien, vorzugsweise von Polyester und insbesondere von Polyestergeweben. Sie ergeben gute Weißeffekte.

Die folgenden Beispiele sollen die Erfindung näher erläutern. Die dort genannten Prozentangaben beziehen sich, sofern nicht anders vermerkt, auf das Gewicht.

Beispiel 1

Zu einer Lösung von 12,9 g 2-Cyano-4'-formylstilben (93 %ig) und 17,5 g 4-(Heptylsulfonyl)-benzylphosphonsäurediethylester in 100 ml N,N-Dimethylformamid ließ man unter starkem Rühren bei 40 °C 9 g methanolische Natriummethanolatlösung (30 %ig) zutropfen. Man rührte 4 Stunden bei 40 °C und 16 Stunden bei 20 bis 24 °C, gab dann 40 ml Wasser hinzu und saugte das ausgefallene Produkt ab. Der Rückstand wurde zuerst mit 50 ml Methanol und dann mit 200 ml Wasser gewaschen. Anschließend trocknete man den Rückstand bei 50 °C unter vermindertem Druck. Man erhielt 15,9 g (76 % d.Th.) des Rohprodukts der Formel

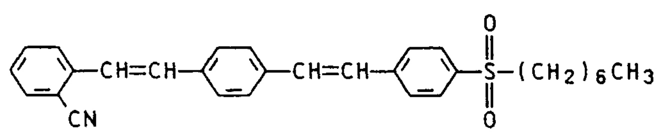

das durch Umkristallisieren aus Toluol gereinigt wurde.

2-Cyano-4'-formylstilben wurde dabei auf folgende Weise hergestellt:

Zu einer Lösung von 201 g Terephthalaldehyd und 447 g o-Cyanobenzylphosphonsäurediethylester (93 %ig) in 2000 ml 2-Methoxyethylacetat ließ man unter starkem Rühren bei 40 bis 45 °C innerhalb von 18 Stunden 270 g methanolische Natriummethanolatlösung (30 %ig) zutropfen. Man rührte 48 Stunden bei 20 bis 25 °C nach, saugte das ausgefallene Produkt ab und wusch gründlich mit Wasser nach. Man erhielt so 271 g der Verbindung der Formel

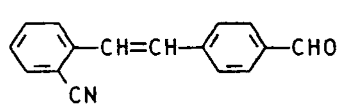

mit dem Schmelzpunkt 143 bis 144 °C.

Den verwendeten 4-(Heptylsulfonyl)benzylphosphonsäurediethylester stellte man auf folgende Weise her:

Zu einer Lösung von 1-Methyl-4-(heptylsulfonyl)benzol in 1000 ml Cyclohexan gab man bei 60 °C

portionsweise eine Mischung aus 95 g N-Bromsuccinimid und 1,3 g Azo-bis-isobutyronitril und rührte noch 4 Stunden unter Rückfluß nach. Danach wurde das resultierende Succinimid abfiltriert, der Filterkuchen mit 100 ml Cyclohexan gewaschen und das Filtrat unter vermindertem Druck eingeengt. Das verbleibende ölige Produkt ist genügend rein und wurde ohne Destillation weiterverarbeitet.

17,3 g 1-Brommethyl-4-(heptylsulfonyl)benzol wurden bei 150 °C zu 31 ml Triethylphosphit getropft. Danach rührte man noch 2 Stunden unter Rückfluß nach und destillierte zunächst im Wasserstrahlvakuum und anschließend im Hochvakuum leichter flüchtige Substanzen ab. Man erhielt so 17,5 g (93 % d.Th.) des Phosphonsäureesters als Rohprodukt.

In analoger Weise werden die in den folgenden Tabellen aufgeführten Verbindungen erhalten. Die $\lambda_{max}$-Werte beziehen sich jeweils auf eine Methylenchloridlösung.

Tabelle 1

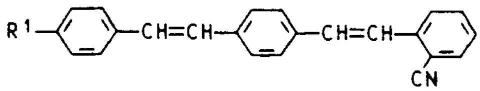

| Verbindung Nr. | $R^1$ | $\lambda_{max}$ /Schmelzpunkt |
|---|---|---|
| 2 | $SO_2(CH_2)_2OCH_3$ | 362 nm |
| 3 | $SO_2(CH_2)_2OCH_2CH_3$ | 362 nm |
| 4 | $SO_2(CH_2)_2O(CH_2)_2CH_3$ | 362 nm |
| 5 | $SO_2(CH_2)_2O(CH_2)_3CH_3$ | 362 nm |
| 6 | $SO_2(CH_2)_3OCH_3$ | 362 nm |
| 7 | $SO_2(CH_2)_3OCH_2CH_3$ | 362 nm |
| 8 | $SO_2(CH_2)_3O(CH_2)_3CH_3$ | 362 nm |
| 9 | $SO_2(CH_2)_2O(CH_2)_2OCH_3$ | 362 nm |
| 10 | $SO_2(CH_2)_2O(CH_2)_2OCH_2CH_3$ | 362 nm |
| 11 | $SO_2(CH_2)_2O(CH_2)_2O(CH_2)_3CH_3$ | 362 nm |
| 12 | 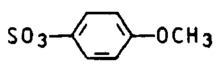 | 369 nm |
| 13 | 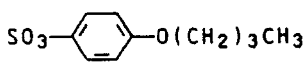 | 369 nm |
| 14 | 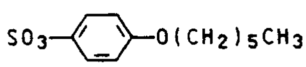 | 369 nm |
| 15 | $SO_2NH(CH_2)_2OCH_3$ | 368 nm |
| 16 | $SO_2NH(CH_2)_2OCH_2CH_3$ | 368 nm |
| 17 | $SO_2NH(CH_2)_2O(CH_2)_2CH_3$ | 368 nm |
| 18 | $SO_2NH(CH_2)_2O(CH_2)_3CH_3$ | 368 nm |
| 19 | $SO_2NH(CH_2)_3OCH_3$ | 368 nm |
| 20 | $SO_2NH(CH_2)_3OCH_2CH_3$ | 368 nm |
| 21 | $SO_2NH(CH_2)_3O(CH_2)_3CH_3$ | 368 nm |
| 22 | $SO_2NH(CH_2)_2O(CH_2)_2OCH_3$ | 368 nm |
| 23 | $SO_2NH(CH_2)_2O(CH_2)_2OCH_2CH_3$ | 368 nm |
| 24 | $SO_2NH(CH_2)_2O(CH_2)_2O(CH_2)_3CH_3$ | 368 nm |
| 25 | $SO_2(CH_2)_2CH_3$ | 153-156$^0$C |
| 26 | $SO_2CH(CH_3)_2$ | 156-160$^0$C |
| 27 | $SO_2CH_2CH(CH_3)_2$ | 107-111$^0$C |
| 28 | $SO_2(CH_2)_4CH_3$ | 126-128$^0$C |
| 29 | $SO_2(CH_2)_5CH_3$ | 131-132$^0$C |

Tabelle 1 (Forts.)

| Verbindung Nr. | $R^1$ | $\lambda_{max}$/Schmelzpunkt |
|---|---|---|
| 30 | $SO_2(CH_2)_6CH_3$ | 128-131°C |
| 31 | $SO_2$-Isooctyl | 117-125°C |
| 32 | $SO_2(CH_2)_2O\underset{\underset{O}{\|}}{C}CH_3$ | 364 nm |
| 33 | $SO_2(CH_2)_2O\underset{\underset{O}{\|}}{C}CH_2CH_3$ | 364 nm |
| 34 | $SO_2(CH_2)_3O\underset{\underset{O}{\|}}{C}CH_3$ | 364 nm |
| 35 | $SO_2(CH_2)_3O\underset{\underset{O}{\|}}{C}CH_2CH_3$ | 364 nm |
| 36 | $SO_2(CH_2)_2O(CH_2)_2O\underset{\underset{O}{\|}}{C}CH_2CH_3$ | 364 nm |
| 37 | $SO_2(CH_2)_2OH$ | 365 nm |
| 38 | $SO_2(CH_2)_3OH$ | 365 nm |
| 39 | $SO_2(CH_2)_6OH$ | 365 nm |
| 40 | $SO_2$— 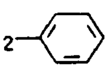 | 233-238°C |

Tabelle 2

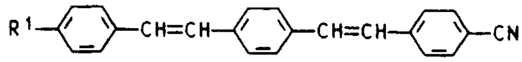

| Verbindung Nr. | $R^1$ | $\lambda_{max}$ /Schmelzpunkt |
|---|---|---|
| 41 | $SO_2NH(CH_2)_2OCH_3$ | 369 nm |
| 42 | $SO_2NH(CH_2)_2OCH_2CH_3$ | 369 nm |
| 43 | $SO_2NH(CH_2)_2O(CH_2)_2CH_3$ | 369 nm |
| 44 | $SO_2NH(CH_2)_2O(CH_2)_3CH_3$ | 369 nm |
| 45 | $SO_2NH(CH_2)_3OCH_3$ | 369 nm |
| 46 | $SO_2NH(CH_2)_3OCH_2CH_3$ | 369 nm |
| 47 | $SO_2NH(CH_2)_3O(CH_2)_3CH_3$ | 369 nm |
| 48 | $SO_2NH(CH_2)_2O(CH_2)_2OCH_3$ | 369 nm |
| 49 | $SO_2NH(CH_2)_2O(CH_2)_2OCH_2CH_3$ | 369 nm |
| 50 | $SO_2NH(CH_2)_2O(CH_2)_2O(CH_2)_3CH_3$ | 369 nm |
| 51 | 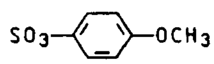 | 369 nm |
| 52 | 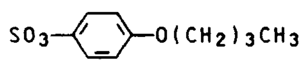 | 369 nm |
| 53 | 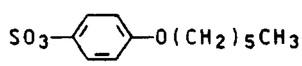 | 369 nm |
| 54 | $SO_2(CH_2)_2OCH_3$ | 364 nm |
| 55 | $SO_2(CH_2)_2OCH_2CH_3$ | 364 nm |
| 56 | $SO_2(CH_2)_2O(CH_2)_2CH_3$ | 364 nm |
| 57 | $SO_2(CH_2)_2O(CH_2)_3CH_3$ | 364 nm |
| 58 | $SO_2(CH_2)_3OCH_3$ | 364 nm |
| 59 | $SO_2(CH_2)_3OCH_2CH_3$ | 364 nm |
| 60 | $SO_2(CH_2)_3O(CH_2)_3CH_3$ | 364 nm |
| 61 | $SO_2(CH_2)_2O(CH_2)_2OCH_3$ | 364 nm |
| 62 | $SO_2(CH_2)_2O(CH_2)_2OCH_2CH_3$ | 364 nm |
| 63 | $SO_2(CH_2)_2O(CH_2)_2O(CH_2)_3CH_3$ | 364 nm |
| 64 | $SO_2(CH_2)_2CH_3$ | 264-275°C |
| 65 | $SO_2CH(CH_3)_2$ | 230-235°C |
| 66 | $SO_2CH_2CH(CH_3)_2$ | 159-160°C |
| 67 | $SO_2(CH_2)_4CH_3$ | 177-179°C |
| 68 | $SO_2(CH_2)_5CH_3$ | 164-166°C |
| 69 | $SO_2(CH_2)_6CH_3$ | 177-179°C |

Tabelle 2 (Forts.)

| Verbindung Nr. | $R^1$ | $\lambda_{max}$ /Schmelzpunkt |
|---|---|---|
| 70 | $SO_2(CH_2)_2O\underset{\underset{O}{\|}}{C}CH_3$ | 364 nm |
| 71 | $SO_2(CH_2)_2O\underset{\underset{O}{\|}}{C}CH_2CH_3$ | 364 nm |
| 72 | $SO_2(CH_2)_3O\underset{\underset{O}{\|}}{C}CH_3$ | 364 nm |
| 73 | $SO_2(CH_2)_3O\underset{\underset{O}{\|}}{C}CH_2CH_3$ | 364 nm |
| 74 | $SO_2(CH_2)_2O(CH_2)_2O\underset{\underset{O}{\|}}{C}CH_2CH_3$ | 364 nm |
| 75 | $SO_2(CH_2)_2OH$ | 365 nm |
| 76 | $SO_2(CH_2)_3OH$ | 365 nm |
| 77 | $SO_2(CH_2)_6OH$ | 365 nm |
| 78 | $SO_2$– 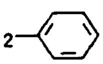 | 256-264°C |

Tabelle 3

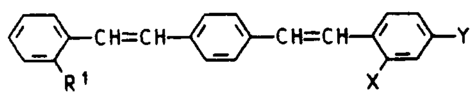

| Verbindung Nr. | $R^1$ | X | Y | $\lambda_{max}$ /Schmelzpunkt |
|---|---|---|---|---|
| 79 | $SO_2(CH_2)_2CH_3$ | H | CN | |
| 80 | $SO_2CH(CH_3)_2$ | H | CN | |
| 81 | $SO_2(CH_2)_4CH_3$ | H | CN | 136-140°C |
| 82 | $SO_2$– 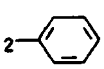 | H | CN | |
| 83 | $SO_2(CH_2)_2CH_3$ | CN | H | |
| 84 | $SO_2CH(CH_3)_2$ | CN | H | |
| 85 | $SO_2(CH_2)_4CH_3$ | CN | H | 95- 99°C |
| 86 | $SO_2$– 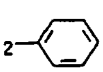 | CN | H | |
| 87 | $SO_3$– 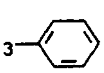 | CN | H | |
| 88 | $SO_2N(C_2H_5)_2$ | CN | H | |

## Patentansprüche

**1.** Bis-styrylverbindungen der Formel I

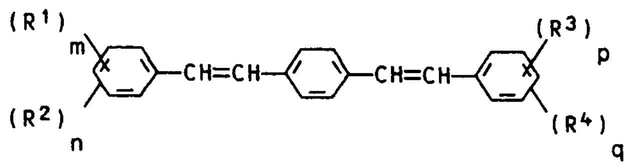

(I)

in der

$R^1$ gegebenenfalls substituiertes Phenoxysulfonyl, $C_1-C_{13}$-Alkylsulfonyl, das gegebenenfalls durch Hydroxy oder $C_1-C_4$-Alkanoyloxysubstituiert ist und dessen Alkylkette durch ein oder mehrere Sauerstoffatome unterbrochen sein kann, $C_1-C_8$-Mono- oder Dialkylsulfamoyl, wobei die Alkylkette jeweils durch ein oder mehrere Sauerstoffatome unterbrochen sein kann, oder gegebenenfalls substituiertes Phenylsulfonyl,

$R^2$ und $R^3$ gleich oder verschieden sind und jeweils unabhängig voneinander Wasserstoff, Fluor, Chlor, $C_1-C_4$-Alkyl, $C_1-C_4$-Alkoxy oder gegebenenfalls substituiertes Phenoxy,

$R^4$ Fluor, Chlor, Cyano, Carbamoyl, $C_1-C_8$-Mono- oder -Dialkylcarbamoyl, deren Alkylkette jeweils durch ein oder mehrere Sauerstoffatome unterbrochen sein kann, Pyrrolidinocarbonyl, Piperidinocarbonyl, Morpholinocarbonyl, Piperazinocarbonyl, N-($C_1-C_4$-Alkyl)piperazinocarbonyl, $C_1-C_8$-Alkoxycarbonyl, dessen Alkylkette durch ein oder mehrere Sauerstoffatome unterbrochen sein kann, oder gegebenenfalls substituiertes Phenoxycarbonyl und

m, n, p und q gleich oder verschieden sind und jeweils unabhängig voneinander 1 oder 2 bedeuten, wobei die Verbindungen der Formeln

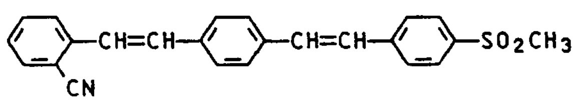 oder $-SO_2C_2H_5$,

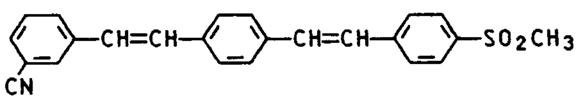 und

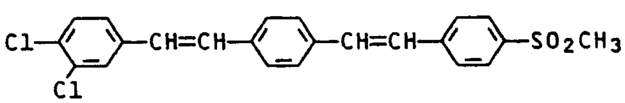

ausgenommen sind.

**2.** Bis-styrylverbindungen gemäß Anspruch 1, dadurch gekennzeichnet, daß

$R^1$ Phenoxysulfonyl, durch $C_1-C_6$-Alkoxy substituiertes Phenoxysulfonyl, $C_2-C_{10}$-Alkylsulfonyl, dessen Alkylkette durch ein oder zwei Sauerstoffatome unterbrochen ist und das gegebenenfalls durch Hydroxy oder $C_1-C_4$-Alkanoyloxy substituiert ist, $C_1-C_8$-Mono- oder Dialkylsulfamoyl, wobei die Alkylkette jeweils durch ein oder zwei Sauerstoffatome unterbrochen sein kann, oder Phenylsulfonyl,

$R^2$ und $R^3$ jeweils Wasserstoff

$R^4$ Chlor, Cyano, $C_1-C_4$-Alkoxycarbonyl, $C_1-C_4$-Dialkylcarbamoyl, Phenoxycarbonyl oder durch Methyl, Ethyl, Methoxy oder Chlor substituiertes Phenoxycarbonyl und

m und q jeweils 1 bedeuten.

**3.** Verwendung der Bis-styrylverbindungen gemäß Anspruch 1 zum optischen Aufhellen von synthetischen, halbsynthetischen oder natürlichen organischen Polymermaterialien.

**Claims**

**1.** A bis-styryl compound of the formula I

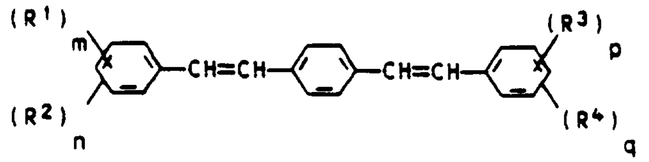

(I)

where

| | |
|---|---|
| $R^1$ | is unsubstituted or substituted phenoxysulfonyl, $C_1-C_{13}$-alkylsulfonyl which is unsubstituted or substituted by hydroxyl or $C_1-C_4$-alkanoyloxy and whose alkyl chain may be interrupted by one or more oxygen atoms, or $C_1-C_8$-mono- or -dialkylsulfamoyl, where the alkyl chain in each case may be interrupted by one or more oxygen atoms, or unsubstituted or substituted phenylsulfonyl, |
| $R^2$ and $R^3$ | are identical or different and, independently of one another, are each hydrogen, fluorine, chlorine, $C_1-C_4$-alkyl, $C_1-C_4$-alkoxy or unsubstituted or substituted phenoxy, |
| $R^4$ | is fluorine, chlorine, cyano, carbamoyl, $C_1-C_8$-mono-or -dialkylcarbamoyl whose alkyl chain in each case may be interrupted by one or more oxygen atoms, or pyrrolidinocarbonyl, piperidinocarbonyl, morpholinocarbonyl, piperazinocarbonyl, N-($C_1-C_4$-alkyl)piperazinocarbonyl, $C_1-C_8$-alkoxycarbonyl whose alkyl chain may be interrupted by one or more oxygen atoms, or unsubstituted or substituted phenoxycarbonyl and |
| m, n, p and q | are identical or different and, independently of one another, are each 1 or 2, with the exception of the compounds of the formulae |

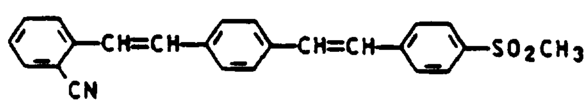

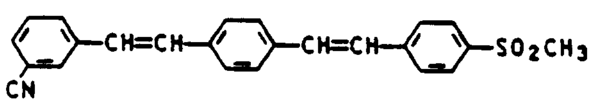

and

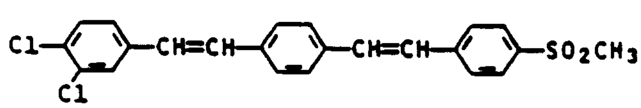

**2.** A bis-styryl compound as claimed in claim 1, wherein

| | |
|---|---|
| $R^1$ | is phenoxysulfonyl, $C_1-C_6$-alkoxy-substituted phenoxysulfonyl, $C_2-C_{10}$-alkylsulfonyl whose alkyl chain is interrupted by one or two oxygen atoms and which is unsubstituted or substituted by hydroxyl or $C_1-C_4$-alkanoyloxy, or $C_1-C_8$-mono- or dialkylsulfamoyl, where the alkyl chain in each case may be interrupted by one or two oxygen atoms, or phenylsulfonyl, |
| $R^2$ and $R^3$ | are each hydrogen, |
| $R^4$ | is chlorine, cyano, $C_1-C_4$-alkoxycarbonyl, $C_1-C_4$-dialkylcarbamoyl, phenoxycarbonyl or methyl-, ethyl-, methoxy- or chlorine-substituted phenoxycarbonyl and |
| m and q | are each 1. |

3. Use of a bis-styryl compound as claimed in claim 1 for the optical brightening of synthetic, cellulosic or natural organic polymer materials.

**Revendications**

1. Composés bis-styrylés de formule I

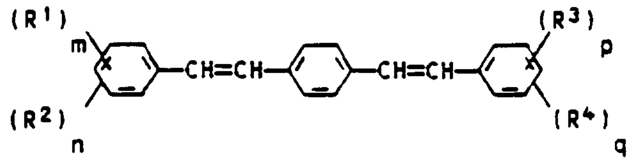

(I)

dans laquelle

- $R^1$ représente un reste phénoxysulfonyle éventuellement substitué, alkylsulfonyle en $C_1-C_{13}$ qui est éventuellement substitué par un groupement hydroxy ou alcanoyloxy en $C_1-C_4$ et dont la chaîne alkyle peut être interrompue par un ou plusieurs atomes d'oxygène, mono ou di(alkyl en $C_1-C_8$)sulfamoyle, la chaîne alkyle pouvant être interrompue chaque fois par un ou plusieurs atomes d'oxygène, ou phénylsulfonyle éventuellement substitué,
- $R^2$ et $R^3$ sont identiques ou différents et représentent chacun, indépendamment l'un de l'autre, un atome d'hydrogène, de fluor, de chlore ou un reste alkyle en $C_1-C_4$, alcoxy en $C_1-C_4$ ou phénoxy éventuellement substitué,
- $R^4$ représente un atome de fluor, de chlore ou un reste cyano, carbamoyle, mono ou di(alkyl en $C_1-C_8$)carbamoyle, dont la chaîne alkyle peut être interrompue chaque fois par un ou plusieurs atomes d'oxygène, pyrrolidinocarbonyle, pipéridinocarbonyle, morpholinocarbonyle, pipérazinocarbonyle, N-(alkyl en $C_1-C_4$)-pipérazinocarbonyle, $C_1-C_8$-alcoxycarbonyle, dont la chaîne alkyle peut être interrompue par un ou plusieurs atomes d'oxygène, ou phénoxycarbonyle éventuellement substitué, et
- m, n, p et q sont identiques ou différents et représentent chacun, indépendamment les uns des autres, le nombre 1 ou 2,

les composés de formules

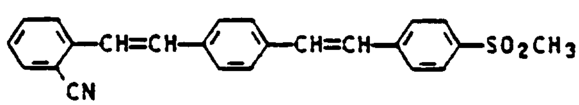

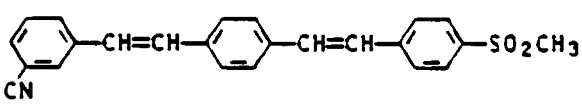

et

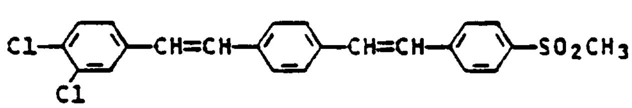

étant exclus.

2. Composés bis-styrylés selon la revendication 1, caractérisés en ce que
   - $R^1$ représente un reste phénoxysulfonyle, phénoxysulfonyle substitué par un groupement alcoxy en $C_1-C_6$, alkylsulfonyle en $C_2-C_{10}$, dont la chaîne alkyle est interrompue par un ou plusieurs atomes d'oxygène et qui est éventuellement substitué par un groupement hydroxy ou alcanoyloxy en $C_1-C_4$, mono ou di(alkyl en $C_1-C_8$)sulfamoyle, la chaîne alkyle pouvant être interrompue chaque fois par un ou plusieurs atomes d'oxygène, ou phénylsulfonyle,

| | |
|---|---|
| $R^2$ et $R^3$ | représentent chacun un atome d'hydrogène, |
| $R^4$ | représente un atome de chlore ou un reste cyano, (alcoxy en $C_1-C_4$)carbonyle, di(alkyl en $C_1-C_4$)carbamoyle, phénoxy-carbonyle ou phénoxycarbonyle substitué par un groupement méthyle, éthyle, méthoxy ou par un atome de chlore, et |
| m et q | représentent chacun le nombre 1. |

3. Utilisation des composés bis-styrylés selon la revendication 1 pour le blanchiment optique de matières polymères organiques synthétiques, semi-synthétiques ou naturelles.